# EUROPÄISCHE PATENTSCHRIFT

(11) **EP 1 030 825 B1**
(45) Veröffentlichungstag und Bekanntmachung des Hinweises auf die Patenterteilung: **29.08.2001**
(21) Anmeldenummer: 98954486.1
(22) Anmeldetag: 12.11.1998
(51) Int. Cl.: C07C 2/10

(54) **VERFAHREN ZUR HERSTELLUNG VON IM WESENTLICHEN UNVERZWEIGTEN OCTENEN UND DODECENEN DURCH OLIGOMERISIERUNG VON UNVERZWEIGTEN BUTENEN**
METHOD FOR PRODUCING ESSENTIALLY UNBRANCHED OCTENES AND DODECENES BY OLIGOMERISING UNBRANCHED BUTENES
PROCEDE DE PRODUCTION D'OCTENES ET DE DODECENES ESSENTIELLEMENT NON RAMIFIES PAR OLIGOMERISATION DE BUTENES NON RAMIFIES

(30) Priorität: 14.11.1997 DE 19750531
(43) Veröffentlichungstag der Anmeldung: 30.08.2000
(73) Patentinhaber: BASF AKTIENGESELLSCHAFT, 67056 Ludwigshafen (DE)
(72) Erfinder: VICARI, Maximilian, D-67117 Limburgerhof (DE); WALTER, Marc, D-67227 Frankenthal (DE); ULONSKA, Armin, D-67112 Mutterstadt (DE); BROX, Wolfgang, D-69118 Heidelberg (DE); KEUSER, Ulrich, D-67273 Weisenheim (DE)
(86) Internationale Anmeldenummer: EP9807028
(87) Internationale Veröffentlichungsnummer: WO9925668

(56) Entgegenhaltungen:
- DE-A- 4 339 713

## Beschreibung

Die Erfindung betrifft die Oligomerisierung von Buten-1 und/oder Buten-2 zu im wesentlichen linearen Octenen und Dodecenen in Gegenwart eines heterogenen Nickel enthaltenden Katalysators, wobei eine Konzentration der gebildeten Oligomeren im Reaktionsgemisch von höchstens 25 Gew.-% eingehalten wird.

Olefine mit 2 bis 6 Kohlenstoffatomen und deren Gemische, insbesondere Olefine mit 4 Kohlenstoffatomen, stehen in großen Mengen sowohl aus FCC-Anlagen als auch aus Steamcrackern zur Verfügung. Der jeweilige C₄-Schnitt, d.h. das Gemisch aus Butenen und Butanen eignet sich nach Abtrennung des Isobutens sehr gut zur Herstellung von Oligomeren, insbesondere Octenen und Dodecenen. Sowohl die Octene als auch Dodecene können nach Hydroformylierung und nachfolgender Hydrierung zu den entsprechenden Alkoholen z.B. für die Herstellung von Weichmachern Verwendung finden.

Für den Einsatz als Weichmacheralkohol spielt der Verzweigungsgrad für die Eigenschaften des Weichmachers eine ausschlaggebende Rolle. Der Verzweigungsgrad wird durch den Iso-Index beschrieben, der die mittlere Zahl der Methylverzweigungen in der jeweiligen Fraktion angibt. So tragen z.B. n-Octene mit 0, Methylheptene mit 1 und Dimethylhexene mit 2 zum Iso-Index einer Fraktion bei. Je niedriger der Iso-Index ist, um so linearer sind die Moleküle in der jeweiligen Fraktion aufgebaut. Je höher die Linearität, d.h. je niedriger der Iso-Index ist, um so höher sind die Ausbeuten in der Oxierung und umso besser sind die Eigenschaften des damit hergestellten Weichmachers.

Die Oligomerisierung wird großtechnisch entweder homogen- oder heterogenkatalytisch ausgeführt.

Die homogenkatalytische Arbeitsweise ist z.B. durch A. Chauvel und G. Lefebvre in Petrochemical Processes, Band 1, Éditions Technip (1989), S. 183 bis 187, eingehend beschrieben.

Nachteil des homogenkatalytischen Verfahrens besteht in der Notwendigkeit, den Katalysator vom Reaktionsgemisch zu trennen. Dieser Abtrennungsschritt verursacht Abfallströme, die aufgrund vor. Umweltauflagen nochmals aufgearbeitet werden müssen. Schließlich läßt sich der homogene Katalysator nicht regenerieren.

Diese Nachteile bestehen dagegen bei der heterogenkatalysierten Olefinoligomerisierung nicht. Ein großtechnisch ausgeübtes heterogenkatalysiertes Verfahren ist das Octol-Verfahren der Firmen UOP und Hüls AG, in dem bei Temperaturen von 30°C bis 250°C-und Drücken von 20 bis 80 bar C₃- und C₄-Olefine an einem Katalysator bestehend, z.B. aus auf SiO₂ als Träger aufgebrachte Phosphorsäure zu höheren Olefinen umgesetzt werden. Dieses Verfahren ist z.B. in Petrochemical Processes a.a.o. und in Hydrocarbon Processing, Februar 1992, Seiten 45/46, beschrieben.

Weiterhin ist aus DE-A 43 39 713 bekannt, daß man die Buten-Oligomerisierung in vorteilhafter Weise und mit sehr guter Selektivität in Bezug auf lineare Produkte durchführen kann, wenn man bestimmte Nickel enthaltende Katalysatoren verwendet.

Während das homogenkatalytische Verfahren die genannten Nachteile bezüglich der Katalysatornutzung hat und das heterogenkatalytische Octol-Verfahren bei Verwendung von sauren Katalysatoren zu wenig linearen Produkten führt, war das Verfahren gemäß DE-A 43 39 713 noch bezüglich der Katalysatorstandzeit und der Selektivität für die Octenbildung verbesserungswürdig.

Es war daher die Aufgabe der Erfindung, ein heterogenkatalytisches Verfahren zu finden, das bei sehr gutem Umsatz und hoher Selektivität bezüglich der Bildung von wenig verzweigten Oligomeren eine lange Katalysatorstandzeit ermöglicht.

Diese Aufgabe wurde erfindungsgemäß gelöst mit einem Verfahren zur Herstellung von im wesentlichen unverzweigten Octenen und Dodecenen durch Oligomerisierung von Buten-1 und/oder Buten-2 und Butan enthaltenden Kohlenwasserstoffströmen, die im wesentlichen von Isobuten frei sind, bei Temperaturen von 30 bis 280°C und einem Druck von 10 bis 300 bar über einem Nickel enthaltenden heterogenen Katalysator, wobei man solche Mengen des von dem Reaktionsgemisch abgetrennten Butans und nicht umgesetzten Butens in die Oligomerisierungsreaktion zurückführt, daß der maximale Gehalt an Oligomeren im umgesetzten Reaktionsgemisch 25 Gew.-%, vorzugsweise 20 Gew.-%, an keiner Stelle des Reaktors oder bei Verwendung einer Reaktorkaskade, an keiner Stelle der Reaktorkaskade übersteigt.

Unter "Oligomeren" werden in dieser Anmeldung Dimere, Trimere und höhere Oligomere von Buten-1 und/oder Buten-2 verstanden.

Es hat sich gezeigt, daß es mit dieser Verfahrensführung möglich ist, die Buten-Oligomerisierung mit einem Umsatz von über 70 % bei gleichzeitiger C₈-Selektivität von über 80 % durchzuführen. Außer den guten Umsatz- und Selektivitätswerten bewirkt die Einhaltung der Konzentrationsgrenze des weiteren eine längere Lebensdauer des Katalysators, da die Bildung höhersiedender Verbindungen unterdrückt wird, die sich auf der Katalysator oberfläche ablagern und damit einen Aktivitätsrückgang bewirken können.

In allgemeiner Form ist zwar schon beim homogenkatalytischen Verfahren und für das Octol-Verfahren eine Rückführung ("C₄-recycle") angegeben, offensichtlich jedoch in geringerem Umfang als im vorliegenden Verfahren und ohne die erfindungsgemäßen vorteilhaften Ergebnisse.

So werden gemäß Literatur Petrochemical Processes, Seite 185 beim Octol-Verfahren die nicht umgesetzten C₃-C₄-Kohlenwasserstoffe (zusammen mit unerwünschten Schwersiedern) zurückgeführt "to serve as diluents and avoid excess conversions that would cause a drop in selectivity". Im Gegensatz dazu werden beim erfindungsgemäßen Verfahren hohe Umsätze und hohe Selektivitäten erhalten.

Auch aus Fig. 1, S. 45, von Hydrocarbon Processing, Februar 1992, geht hervor, daß eine C₄-Rückführung durchgeführt wurde. Es ist aber offensichtlich, daß die Rückführung nur in geringem Umfang durchgeführt wurde, d.h. in Mengen, die zu Konzentrationen von über 25 Gew.-% Oligomeren im Reaktionsgemisch führen mußten, da die Reaktion quasi-isotherm durchgeführt wird, während die erfindungsgemäß relativ große zurückzuführende Flüssiggasmenge eine technisch einfachere quasi-adiabatische Arbeitsweise erlaubt.

Als Katalysatoren kommen allgemein an sich bekannte, ein geringe Verzweigung bewirkende, Nickel enthaltende Katalysatoren in Betracht, wie sie z.B. von O'Connor et al in Catalysis Today, 6, 329 (1990), insbesondere auf den Seiten 336-338 und in den in DE-A 4 339 713 zum Stand der Technik zitierten Literaturstellen beschrieben sind, so daß auf die dort angegebenen Fundstellen ausdrücklich Bezug genommen wird. Beispielhaft sind in diesem Zusammenhang Nickel auf Siliciumdioxid, Nickel auf Siliciumdioxid-Aluminiumoxid, Nickel auf Siliciumdioxid-Aluminiumoxid-Schichtsilikaten, wie Glimmer und Tonerden, insbesondere Montmorillonite, Nickel auf Zeolith-Trägern, wie Mordenit, Faujasit, Zeolith X, Zeolith Y, Zeolith ZSM-5 oder andere Zeolithe vom ZSM-Typ, Zeolithe mit MCM-41 Struktur oder CZS-1 Struktur, weiterhin Nickel auf Aluminiumoxid, Nickel auf Zirkoniumoxid, das mit Säuren, wie Schwefelsäure, Phosphorsäure oder Borsäure behandelt ist, NiO₂/ZrO₂/SO₄/SiO₂, sowie Nickel auf sulfatiertem Titandioxid als Katalysatoren zu nennen, wobei das Nickel in diesen Katalysatoren in der Regel in Form von NiO vorliegt.

Nach einer bevorzugten Ausführungsform des erfindungsgemäßen Verfahrens wird die Oligomerisierung in flüssiger Phase durchgeführt unter Verwendung der in DE-A 43 39 713 beschriebenen und beanspruchten Katalysatoren. Deshalb wird auf diese Patentschrift ausdrücklich Bezug genommen und deren Angaben sollen als hier inkorporiert gelten.

Die dort beschriebenen Katalysatoren bestehen in ihren katalytisch aktiven Masse im wesentlichen, d.h. ohne Berücksichtigung von Verunreinigungen, die durch Ausgangs- oder Prozeßchemikalien bei der Katalysatorherstellung eingeschleppt werden, aus Nickeloxid, Siliciumdioxid, Titandioxid und/oder Zirkondioxid sowie gegebenenfalls Aluminiumoxid. Diese Katalysatoren enthalten 10 bis 70 Gew.-% Nickeloxid, 5 bis 30 Gew.-% Titandioxid und/oder Zirkondioxid, 0 bis 20 Gew.-% Aluminiumoxid und als Rest bis zu 100 Gew.-% Siliciumdioxid und sind durch Fällung der Katalysatormasse bei pH 5 bis 9 durch Zugabe einer Nickelnitrat enthaltenden wäßrigen Lösung zu einer Alkaliwasserglaslösung, die Titandioxid und/oder Zirkondioxid enthält, Filtrieren, Trocknen und Tempern bei 350 bis 650°C, erhältlich.

Die Katalysatoren sind bevorzugt in einem Festbett angeordnet und deshalb vorzugsweise in stückiger Form, z.B. in Form von Tabletten (5 mm x 5 mm, 5 mm x 3 mm, 3 mm x 3 mm), Ringen (7 mm x 7 mm x 3 mm, 5 mm x 5 mm x 2 mm, 5 mm x 2 mm x 2 mm) oder Strängen (1,5 mm-Durchmesser, 3 mm-Durchmesser, 5 mm-Durchmesser) verwendet. Die vorstehenden Größenangaben sind lediglich beispielhaft und stellen keine Einschränkung des Gegenstandes der vorliegenden Erfindung dar.

Im einzelnen führt man das erfindungsgemäße Verfahren so aus, daß man einen Buten-1, Buten-2, Butan und höchstens geringe Mengen Isobuten enthaltenden Kohlenwasserstoffstrom, bevorzugt in flüssiger Phase über den beispielhaft genannten NiO enthaltenden Katalysatoren umsetzt.

Geeignete C₄-Kohlenwasserströme sind z.B. Gemische mit folgender Zusammensetzung:

| | |
|---|---|
| Butan | 10 bis 90 Gew.-% |
| Buten | 10 bis 90 Gew.-%, |

wobei die Buten-Fraktion folgende Zusammensetzung haben kann:

| | |
|---|---|
| Buten-1 | 1 bis 50 Gew.-% |
| Buten-2 cis | 1 bis 50 Gew.-% |
| Buten-2 trans | 1 bis 99 Gew.-% |
| iso-Buten | 1 bis 5 Gew.-% |

Als besonders bevorzugter Einsatzstoff wird das sogenannte Raffinat II verwendet, also ein Buten-haltiges C₄-Kohlenwasserstoffgemisch, wie es aus dem C₄-Schnitt von Crackern nach Abtrennung höher ungesättigter Kohlenwasserstoffe, wie Diolefinen, insbesondere 1,3-Butadien, oder Acetylen und nachfolgender Abtrennung des darin enthaltenen Isobutens, erhalten wird. Eine typische Zusammensetzung für ein Raffinat II ist z.B.:

| | |
|---|---|
| i-, n-Butan | 26 Gew.-% |
| i-Buten | 1 Gew.-% |
| Buten-1 | 26 Gew.-% |
| Buten-2-trans | 31 Gew.-% |
| Buten-2-cis | 16 Gew.-% |

Die C₄-Kohlenwasserstoffströme können z.B. in an sich aus der DE-A 39 14 817 bekannter Weise von Butadien, schwefelhaltigen und sauerstoffhaltigen Verbindungen, wie Alkoholen, Aldehyden, Ketonen oder Ethern, durch selektive Hydrierung bzw. Absorption an einem Molekularsieb befreit werden.

Die Oligomerisierungsreaktion findet im allgemeinen bei Temperaturen von 30 bis 280°C, vorzugsweise von 30 bis 140°C und insbesondere von 40 bis 130°C und einem Druck von im allgemeinen 10 bis 300 bar, vorzugsweise von 15 bis 100 bar und insbesondere von 20 bis 70 bar statt. Der Druck wird dabei zweckmäßig so ausgewählt, daß bei der eingestellten Temperatur das Einsatzkohlenwasserstoffgemisch flüssig oder im überkritischen Zustand vorliegt. Der Reaktor ist in der Regel ein mit dem Katalysator beschickter zylindrischer Reaktor, der von dem flüssigen Reaktionsgemisch z.B. von oben nach unten durchströmt wird. Das erfindungsgemäße Oligomerisierungsverfahren kann in einem einzelnen Reaktor bis zum gewünschten Endumsatz der Butene durchgeführt werden, wobei der Oligomerisierungskatalysator in einem einzigen oder mehreren Festbetten im Reaktor angeordnet sein kann. Alternativ kann zur Durchführung des erfindungsgemäßen Verfahrens eine Reaktorkaskade aus mehreren, vorzugsweise zwei, hintereinander geschalteten Reaktoren eingesetzt werden, wobei beim Passieren des bzw. der dem letzten Reaktor der Kaskade vorgeschalteten Reaktors bzw. Reaktoren die Oligomerisierung der Butene im Reaktionsgemisch nur bis zu einem Teilumsatz betrieben wird und der gewünschte Endumsatz erst beim Passieren des Reaktionsgemisches durch den letzten Reaktor der Kaskade erzielt wird. In den einzelnen Reaktoren der Reaktorkaskade kann der Oligomerisierungskatalysator in einem einzigen oder in mehreren Katalysatorfestbetten angeordnet sein.

Weiterhin können in den einzelnen Reaktoren der Reaktorkaskade unterschiedliche Reaktionsbedingungen hinsichtlich Druck und/oder Temperatur im Rahmen der obengenannten Druck- und Temperaturbereiche eingestellt werden. Außerdem ist es möglich, in den einzelnen Reaktoren der Kaskade unterschiedliche Oligomerisierungskatalysatoren einzusetzen, obgleich die Anwendung des gleichen Katalysators in sämtlichen Reaktoren der Kaskade bevorzugt-wird. Für den Fall des Einsatzes eines einzigen Oligomerisierungsreaktors als auch für den Fall der Verwendung einer Reaktorkaskade, in deren Reaktoren der gleiche Oligomerisierungskatalysator eingesetzt wird, wird nachfolgend von einer einstufigen Reaktionszone gesprochen, falls in den einzelnen Reaktoren der Kaskade unterschiedliche Oligomerisierungskatalysatoren angewandt werden, wird dies nachfolgend als mehrstufige Reaktionszone bezeichnet.

Wird ein einziger Oligomerisierungsreaktor verwendet, wird das rückgeführte an Butenen verarmte, vorwiegend Butane enthaltende C₄-Kohlenwasserstoffgemisch, dem Einsatzkohlenwasserstoffgemisch vorteilhaft vor dessen Eintritt in den Reaktor zugemischt - es ist aber auch möglich das Einsatzkohlenwasserstoffgemisch und das rückgeführte C₄-Kohlenwasserstoffgemisch über getrennte Leitungen in den Oligomerisierungsreaktor einzuleiten. Ist der Katalysator im Oligomerisierungsreaktor in mehreren Festbetten angeordnet, kann der rückgeführte Kohlenwasserstoffstrom aufgeteilt und an mehreren Stellen, z.B. vor dem ersten Festbett in Fließrichtung des Reaktionsgemisches und/oder zwischen den einzelnen Katalysatorfestbetten, in den Reaktor eingeleitet werden. Entsprechendes gilt bei Verwendung einer Reaktorkaskade, wobei der rückgeführte Kohlenwasserstoffstrom sowohl vollständig dem ersten Reaktor der Kaskade zugeführt werden kann oder über mehrere Zuleitungen auf die einzelnen Reaktoren der Kaskade, wie für den Fall des Einzelreaktors beschrieben, verteilt werden kann. Nach dem Verlassen der ein- oder mehrstufigen Reaktionszone werden die gebildeten Oligomere in an sich bekannter Weise von den nicht umgesetzten C₄-Kohlenwasserstoffen getrennt und diese C₄-Kohlenwasserstoffe vollständig oder zum größten Teil zurückgeführt, in jedem Fall in einer solchen Menge, daß der Gehalt an Oligomeren im umgesetzten Reaktionsgemisch 25 Gew.-%, vorzugsweise 20 Gew.-%, an keiner Stelle im Reaktor oder bei Verwendung einer Reaktorkaskade, an keiner Stelle der Reaktorkaskade übersteigt. Mit anderen Worten ausgedrückt ist die Rückführung der C₄-Kohlenwasserstoffgemisches so zu steuern, daß der Oligomerengehalt des umgesetzten Reaktionsgemisches an keiner Stelle des Reaktors bzw. im Falle der Anwendung einer Reaktorkaskade an keiner Stelle der Reaktorkaskade 25 Gew.-%, vorzugsweise 20 Gew.-% übersteigt und der Oligomerengehalt im umgesetzten Reaktionsgemisch bei dessen Austritt aus dem Reaktor bzw. im Falle der Verwendung einer Reaktorkaskade, bei dessen Austritt aus der Reaktorkaskade vorteilhafterweise 10 Gew.-% nicht unterschreitet. Zur Erzielung eines solchen Oligomerengehaltes der umgesetzten Reaktionsmischung wird in der Regel ein Gewichtsverhältnis von Rückführstrom zu frisch zugeführtem Einsatzkohlenwasserstoffstrom von 0,5 bis 10, vorzugsweise von 1 bis 7, insbesondere von 1 bis 4 eingestellt, wobei sich diese Angaben auf den stationären Zustand des Reaktionssystems beziehen.

Nach unten besteht an sich keine Grenze für den Oligomerengehalt im Reaktionsgemisch, doch wird das Verfahren bei der Wahl eines sehr geringen Oligomerengehaltes wegen des übermäßig groß werdenden Rückführstromes unwirtschaftlich. Deshalb wird in der Regel eine untere Grenze von 10 Gew.-% Oligomeren, im umgesetzten Reaktionsgemisch vor dessen Aufarbeitung nicht unterschritten.

Unter adiabatischer Reaktionsführung wird im Unterschied zur isothermen Reaktionsführung, bei der die in einer exothermen Reaktion entstehende Wärmemenge durch Kühlung mittels Kühl- oder Thermostatisiervorrichtungen, wie Thermostatisierbäder, Kühlmäntel oder Wärmetauscher, abgeführt wird und so die Temperatur im Reaktor konstant, d.h. isotherm, gehalten wird, eine Betriebsweise verstanden, bei der die in einer exothermen Reaktion freiwerdende Wärmemenge von der Reaktionsmischung im Reaktor aufgenommen und keine Kühlung durch Kühlvorrichtungen angewandt wird. Es versteht sich von selbst, daß eine rein adiabatische Reaktionsführung im theoretisch-akademischen Sinne des Begriffs "adiabatisch" technisch nur mit unwirtschaftlich hohem Aufwand bewerkstelligt werden könnte, da ein - wenn auch vernachlässigbar kleiner - Teil der bei der exothermen Reaktion freiwerdenden Wärmemenge praktisch unvermeidlich auch vom Reaktorkörper aufgenommen und durch Wärmeleitung und Wärmeabstrahlung an die Umwelt abgegeben wird. Im technischen Sinne wird deshalb unter einer adiabatischen Reaktionsführung oder Betriebsweise eine Reaktionsführung oder Betriebsweise verstanden, bei der, abgesehen von dem durch natürliche Wärmeleitung und Wärmeabstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird. Synonym zur adiabatischen Reaktionsführung im technischen Sinne wird deshalb auch der Begriff der "quasi-adiabatischen" Reaktionsführung verwendet. Die Maßnahme einer adiabatischen Reaktionsführung wie sie bei der vorliegenden Erfindung angewandt wird, entspricht also einer adiabatischen Reaktionsführung im vorstehend erläuterten technischen Sinne. Im Unterschied zu dieser adiabatischen Reaktionsführung wird bei der isothermen Reaktionsführung im technischen Sinne der Austrag der Reaktionswärme aus dem Reaktor mittels Kühl- oder Thermostatisiervorrichtungen über das durch natürliche Wärmeleitung oder Wärmeabstrahlung gegebene Maß hinaus gezielt forciert, wobei die Reaktionswärme in der Regel zunächst von einem Wärmeträgermedium, dem Kühlmittel, aufgenommen wird, bevor sie an die Umgebung abgegeben oder, beispielsweise bei Verwendung von Wärmetauchern, zur Erwärmung von Stoffen oder zur Energiegewinnung, genutzt wird. Selbstverständlich ist eine isotherme Reaktionsführung im technischen Sinne von einer isothermen Reaktionsführung im theoretisch-akademischen Sinne zu unterscheiden, da ein - wenn auch vernachlässigbar kleiner - Teil der Reaktionswärme praktisch unvermeidlich mit dem erwärmten Reaktionsgemisch ausgetragen wird, weshalb auch im Falle der isothermen Reaktionsführung im technischen Sinne synonym von einer "quasi-isothermen" Reaktionsführung gesprochen wird.

Da die exotherme Reaktion im Fall des vorliegenden Oligomerisierungsverfahrens alleine durch den Kontakt der Butene mit dem Oligomerisierungskatalysator zustande kommt und somit nur im Bereich der Katalysatorschüttung Wärme freigesetzt wird, kann die Reaktionstemperatur in der Katalysatorschüttung und somit auch die Temperatur im Reaktor im Prinzip durch die Zufuhr der Reaktanden gesteuert werden. Je mehr Buten am Katalysator umgesetzt wird, umso mehr erhöht sich die Temperatur in der Katalysatorschüttung, d.h. umso höher wird die Reaktionstemperatur. Da bei einer solchen adiabatischen Betriebsweise keine Wärme über Kühlvorrichtungen abgeführt wird, wird die bei der Oligomerisierung entstehende Reaktionswärme praktisch alleine durch die die Katalysatorschüttung durchströmende Reaktionsmischung abgeführt. Eine solche Betriebsweise würde ohne zusätzliche Maßnahmen bei Verwendung der erfindungsgemäß einzusetzenden Eingangskohlenwasserstoffgemisches den Durchsatz und somit den Umsatz pro Zeiteinheit, bezogen auf eine Volumeneinheit des eingesetzten Oligomerisierungskatalysators, stark limitieren, da durch eine Erhöhung des Umsatzes die Reaktionstemperatur stark ansteigen würde, was eine erhöhte Bildung von Nebenprodukten zur Folge hätte und im schlimmsten Fall zu einem unkontrollierten Durchgehen der Reaktion mit der Folge einer Polymerisation der Butene und einer Überhitzung und irreversiblen Schädigung des Katalysators führen könnte.

Es wurde nun gefunden, daß durch die zuvor geschilderte Maßnahme der Rückführung der vom Oligomerenprodukt abgetrennten, nicht-umgesetzten C₄-Kohlenwasserstoffe in den Oligomerisierungsreaktor bzw. gegebenenfalls in die zur Oligomerisierung verwendete Reaktorkaskade, die Reaktionstemperatur in der Katalysatorschüttung auch bei hohem Umsatz der Butene im gewünschten Temperaturbereich gehalten werden und eine hohe Selektivität bezüglich der gewünschten Oligomerisierungsprodukte erzielt werden kann. Da der in die Oligomerisierungsreaktion zurückgeführte Strom nicht-umgesetzter C₄-Kohlenwasserstoffe einen geringeren Gehalt an reaktiven Butenen und einen höheren Gehalt an unter den Reaktionsbedingungen inerten Butanen hat als der dem Oligomerisierungsreaktor frisch zugeführte Einsatzkohlenwasserstoffstrom, bewirkt der diesem Strom zugemischte Rückführstrom sozusagen dessen Verdünnung mit inerten Butanen, die zusätzlich zur Abführung der Reaktionswärme aus der Katalysatorschüttung beitragen. Somit kann die Oligomerisierung im Oligomerisierungsreaktor unter adiabatischen Bedingungen, d.h. ohne Verwendung von Kühlvorrichtungen im Reaktor, wie sie im Falle einer isothermen Betriebsweise erforderlich sind, durch die Abführung der Reaktionswärme mit dem Reaktionsgemisch durchgeführt werden.

Es ist also eine Besonderheit der erfindungsgemäßen Reaktionsführung, daß das Verfahren quasi-adiabatisch ausgeübt werden kann, da die Wärmetönung im Reaktor durch das Verdünnen der Butene mit dem zurückgeführten Kohlenwasserstoffstrom und durch die Wahl der Menge und Temperatur dieses Stroms beliebig kontrolliert werden kann. Der rückgeführte Kohlenwasserstoffstrom kann vor seiner Zumischung zum frisch zugeführten Einsatzkohlenwasserstoffstrom oder im Falle einer direkten Einleitung in den Oligomerisierungsreaktor, vor seiner Einleitung, auf eine tiefere Temperatur abgekühlt worden sein, wodurch die Abführung der Reaktionswärme zusätzlich verbessert werden kann. Die quasi-adiabatische Betriebsweise umfaßt auch eine Verfahrenskonfiguration des erfindungsgemäßen Verfahrens, bei der die Umsetzung der Butene zu Oligomeren in einer Reaktorkaskade aus zwei oder mehreren, vorzugsweise zwei, Oligomerisierungsreaktoren verteilt wird und das teilumgesetzte Reaktionsgemisch nach Verlassen des einen Reaktors und vor Eintritt in den nachfolgenden Reaktor der Kaskade mittels herkömmlicher Kühlvorrichtungen, wie Kühlmäntel oder Wärmetauscher, gekühlt wird. Die adiabatische Fahrweise führt im Vergleich zu einem isotherm betriebenen Verfahren zu einem beträchtlichen Herabsetzen der Apparate-Investitionskosten.

Durch die Rückführung der nicht umgesetzten Butene mit dem Rückführstrom in den Oligomerisierungsreaktor wird eine hohe Ausnutzung der mit dem Einsatzkohlenwasserstrom dem Oligomerisierungsreaktor zugeführten Butene für die Oligomerisierung erzielt. Weiterhin ermöglicht die quasi-adiabatische Betriebsweise einen hohen Butenumsatz bei gleichzeitig hoher Selektivität.

Die Auftrennung der Oligomeren erfolgt in an sich bekannter Weise durch fraktionierte Destillation, vor allem in die gewünschten Octen- und Dodecenfraktionen, die als Ausgangsmaterialien für die Hydroformylierung zur Herstellung der Weichmacheralkohole Nonanol und Tridecanol dienen. Aufgrund der geringen Verzweigung der Ausgangsoligomeren weisen die daraus hergestellten höheren Alkohole hervorragende Eigenschaften für die Herstellung von Weichmachern auf.

In den folgenden Beispielen ist das erfindungsgemäße Verfahren im einzelnen demonstriert.

### Beispiele

### Beispiele 1 bis 4

Die Versuche wurden in einem isothermen Reaktor (Länge ca. 1,5 m, Durchmesser 30 mm) bei 20 bar und 80°C kontinuierlich durchgeführt. Eingesetzt wurde ein Raffinat II mit folgender Zusammensetzung:

| | |
|---|---|
| i-Butan | 3 Gew.-% |
| n-Butan | 15 Gew.-% |
| i-Buten | 2 Gew.-% |
| Buten-1 | 30 Gew.-% |
| Buten-2-trans | 32 Gew.-% |
| Buten-2-cis | 18 Gew.-% |

Als Katalysator diente ein Material, das gemäß DE-A 43 39 713 hergestellt und zu 5 mm x 5 mm Tabletten verformt worden war. Zusammensetzung in Gew.-% der Aktivkomponenten: 50 Gew.-% NiO, 12,5 Gew.-% TiO₂, 33,5 Gew.-% SiO₂, 4 Gew.-% Al₂O₃.

Folgende Ergebnisse wurden erzielt (nach Erreichen des stationären Zustands der Umsetzung):

| Beispiel | 1 (Vergl.) | 2 | 3 | 4 |
|---|---|---|---|---|
| Durchsatz (kg_{RaffII}/ (l_{Kat}·h)) | 0,75 | 0,75 | 0,5 | 0,375 |
| C₄-Rückführung/Raff.II (Gew./Gew.) | 0 | 1 | 1,5 | 2 |
| [C₈⁺]_{Reaktoraustritt} (Gew.-%) | 42,6 | 24,3 | 22,3 | 20,0 |
| C₄-Umsatz bezogen auf die im Raff.II enthaltenen Butene (Gew.-%) | 52,0 | 59,2 | 67,9 | 73,1 |
| Selektivität (Gew.-%) | | | | |
| C₈ | 76,9 | 81,7 | 81,5 | 81,4 |
| C₁₂ | 18,4 | 14,8 | 14,9 | 15,1 |
| C₁₆⁺ | 4,7 | 3,5 | 3,6 | 3,5 |
| C₈-Iso-Index | 0,97 | 0,95 | 0,94 | 0,93 |

### Beispiel 5

Die Versuche wurden in eine Reaktorkaskade, bestehend aus zwei adiabatisch betriebenen Reaktoren mit Zwischenkühlung (Länge 2 x ca. 4 m, Durchmesser: 80 cm) bei 30 bar kontinuierlich durchgeführt. Eingesetzt wurde ein Raffinat II mit folgender Zusammensetzung:

| | |
|---|---|
| i-Butan | 2 Gew.-% |
| n-Butan | 10 Gew.-% |
| i-Buten | 2 Gew.-% |
| Buten-1 | 32 Gew.-% |
| Buten-2-trans | 37 Gew.-% |
| Buten-2-cis | 17 Gew.-% |

Als Katalysator diente ein Material, das gemäß DE-A 43 39 713 hergestellt und zu 5 mm x 5 mm Tabletten verformt worden war: Zusammensetzung in Gew.-% der Aktivkomponenten: 50 Gew.-% NiO, 12,5 Gew.-% TiO₂, 33,5 Gew.-% SiO₂, 4 Gew.-% Al₂O₃.

Folgende Ergebnisse wurden erzielt (nach Erreichen des stationären Betriebszustandes):

| | |
|---|---|
| Durchsatz (kg_{RaffII}/(l_{Kat}·h) | 0,375 |
| C₄-Rückführung/Raffinat II (Gew./Gew.) | 3 |
| Eintrittstemperatur 1. Reaktor (°C) | 38 |
| Austrittstemperatur 1. Reaktor (°C) | 67 |
| Eintrittstemperatur 2. Reaktor (°C) | 60 |
| Austrittstemperatur 2. Reaktor (°C) | 75 |
| [C₈⁺]_{Reaktoraustritt} (Gew.-%) | 18,3 |
| C₄-Umsatz bezogen auf die im Raffinat II enthaltenen Butene (Gew.-%) | 83,1 |
| C₈-Selektivität (Gew.-%) | 83,3 |
| C₈-Iso-Index | 1,00 |

## Patentansprüche

1. Verfahren zur Herstellung von im wesentlichen unverzweigten Octenen und Dodecenen durch Oligomerisierung von Buten-1 und/ oder Buten-2 und Butan enthaltende Kohlenwasserstoffströmen, die im wesentlichen von Isobuten frei sind, bei Temperaturen von 30 bis 280°C und einem Druck von 10 bis 300 bar über einem Nickel enthaltenden heterogenen Katalysator, dadurch gekennzeichnet, daß man solche Mengen des von dem Reaktionsgemisch abgetrennten Butans und nicht umgesetzten Butens in die Oligomerisierungsreaktion zurückführt, daß der maximale Gehalt an Oligomeren im umgesetzten Reaktionsgemisch 25 Gew.-% an keiner Stelle im Reaktor oder bei Verwendung einer Reaktorkaskade, an keiner Stelle der Reaktorkaskade übersteigt.

2. Verfahren gemäß Anspruch 1, dadurch gekennzeichnet, daß man die Oligomerisierung in flüssiger Phase durchführt.

3. Verfahren gemäß Anspruch 1, dadurch gekennzeichnet, daß der Gehalt an Oligomeren im umgesetzten Reaktionsgemisch 25 Gew.-% an keiner Stelle im Reaktor oder bei Verwendung einer Reaktorkaskade, an keiner Stelle der Reaktorkaskade, übersteigt und der Oligomerengehalt im umgesetzten Reaktionsgemisch bei dessen Austritt aus dem Reaktor oder bei Verwendung einer Reaktorkaskade, bei dessen Austritt aus der Reaktorkaskade 10 Gew.-% nicht unterschreitet.

4. Verfahren gemäß Anspruch 1, dadurch gekennzeichnet, daß durch die Wahl der Menge und Temperatur des Rückführstroms die Reaktionswärme aufgenommen und die Oligomerisierung adiabatisch oder im wesentlichen adiabatisch ausgeführt wird.

5. Verfahren gemäß Anspruch 1, dadurch gekennzeichnet, daß man die Oligomerisierung über einem Festbettkatalysator durchführt, der im wesentlichen aus NiO, SiO₂, TiO₂ und/oder ZrO₂, sowie gegebenenfalls Al₂O₃ besteht.

6. Verfahren gemäß Anspruch 1, dadurch gekennzeichnet, daß man die Oligomerisierung über einem Festbettkatalysator durchführt, der im wesentlichen aus 10 bis 70 Gew.-% NiO, 5 bis 30 Gew.-% TiO₂ und/oder ZrO₂, 0 bis 20 Gew.-% Al₂O₃ und als Rest zu 100 Gew.-% aus SiO₂ besteht.

7. Verfahren gemäß Anspruch 1, dadurch gekennzeichnet, daß man die Oligomerisierung in einer Reaktorkaskade durchführt.

## Claims

1. A process for preparing essentially unbranched octenes and dodecenes by oligomerization of hydrocarbon streams which comprise 1-butene and/or 2-butene and butane and are essentially free of isobutene, at from 30 to 280°C and a pressure of from 10 to 300 bar over a nickel-containing heterogeneous catalyst, wherein the butane and unreacted butene separated from the reaction mixture are recirculated to the oligomerization reaction in such amounts that the maximum content of oligomers in the converted reaction mixture does not exceed 25 % by weight at any point in the reactor or, if a reactor cascade is used, at any point of the reactor cascade.

2. A process as claimed in claim 1, wherein the oligomerization is carried out in the liquid phase.

3. A process as claimed in claim 1, wherein the content of oligomers in the converted reaction mixture does not exceed 25 % by weight at any point in the reactor or, if a reactor cascade is used, at any point of the reactor cascade and the oligomer content of the converted reaction mixture on leaving the reactor or, if a reactor cascade is used, on leaving the reactor cascade is not less than 10 % by weight.

4. A process as claimed in claim 1, wherein the amount and temperature of the return stream are selected so that the heat of the reaction is taken up and the oligomerization is carried out adiabatically or essentially adiabatically.

5. A process as claimed in claim 1, wherein the oligomerization is carried out over a fixed-bed catalyst consisting essentially of NiO, SiO₂, TiO₂ and/or ZrO₂ and, if desired, Al₂O₃.

6. A process as claimed in claim 1, wherein the oligomerization is carried out over a fixed-bed catalyst consisting essentially of from 10 to 70 % by weight of NiO, from 5 to 30% by weight of TiO₂ and/or ZrO₂, of from 0 to 20 % by weight of Al₂O₃ and of SiO₂ as the balance to 100 % by weight.

7. A process as claimed in claim 1, wherein the oligomerization is carried out in a reactor cascade.

## Revendications

1. Procédé pour la fabrication des octènes et des dodécènes en majorité non ramifiés, par oligomérisation du butène-1 et/ou du butène-2 et des courants d'hydrocarbures contenants du butane et essentiellement exempts d'isobutène, à des températures de 30°C à 280°C et sous une pression de 10 à 300 bars, sur un catalyseur hétérogène contenant du nickel, caractérisé en ce que l'on recycle dans la réaction de l'oligomérisation le butène n'ayant pas réagi et le butène séparé du mélange réactionnel, en quantités choisies de telle manière que la teneur maximale en oligomères dans le mélange réactionnel ayant réagi, ne dépasse 25% en poids à aucun endroit dans le réacteur, ou, lors de l'emploi d'une cascade de réacteurs, à aucun endroit dans la cascade de réacteurs.

2. Procédé selon la revendication 1, caractérisé en ce que l'on réalise l'oligomérisation en phase liquide.

3. Procédé selon la revendication 1, caractérisé en ce que la teneur en oligomères, dans le mélange réactionnel ayant réagi, ne dépasse 25% en poids à aucun endroit dans le réacteur, ou, lors de l'emploi d'une cascade de réacteurs, à aucun endroit dans la cascade de réacteurs, et que la teneur en oligomères ne soit pas inférieur à 10% en poids dans le mélange réactionnel ayant réagi au moment de sa sortie du réacteur, ou, lors de l'emploi d'une cascade de réacteurs, au moment de sa sortie de la cascade de réacteurs.

4. Procédé selon la revendication 1, caractérisé en ce que la chaleur de réaction est absorbée en réglant la quantité et la température du courant recyclé, et que l'oligomérisation est réalisée de manière adiabatique ou essentiellement adiabatique.

5. Procédé selon la revendication 1, caractérisé en ce que l'on réalise l'oligomérisation sur un catalyseur à lit fixe qui se compose essentiellement de NiO, SiO₂, TiO₂ et/ou ZrO₂, ainsi qu'éventuellement Al₂O₃.

6. Procédé selon la revendication 1, caractérisé en ce que l'on réalise l'oligomérisation sur un catalyseur à lit fixe qui se compose essentiellement de NiO à raison de 10 à 70% en poids, de TiO₂ et/ou ZrO₂ à raison de 5 à 30% en poids, de Al₂O₃ à raison de 0 à 20% en poids et de SiO₂ en quantité suffisante pour faire 100%.

7. Procédé selon la revendication 1, caractérisé en ce que l'on réalise l'oligomérisation dans une cascade de réacteurs.
